# EUROPÄISCHE PATENTSCHRIFT

(11) **EP 2 481 445 B1**
(45) Veröffentlichungstag und Bekanntmachung des Hinweises auf die Patenterteilung: **30.11.2016**
(21) Anmeldenummer: 11000629.3
(22) Anmeldetag: 26.01.2011
(51) Int. Cl.: A61M 39/22

(54) **Ventil an einer katheter- oder schleusenartigen medizinischen Einrichtung**
Valve on a catheter or sheath-like medical device
Soupape sur un dispositif médical de type cathéter ou écluse

(43) Veröffentlichungstag der Anmeldung: 01.08.2012
(73) Patentinhaber: Geller, Johann-Christoph, Prof. Dr. med., 99425 Weimar (DE)
(72) Erfinder: Geller, Johann-Christoph, Prof. Dr. med., 99425 Weimar (DE)
(74) Vertreter: Schmidt, Axel

(56) Entgegenhaltungen:
- EP-A1- 2 168 628
- WO-A2-2006/025054
- DE-A1-102007 049 126

## Beschreibung

Die Erfindung betrifft ein Ventil mit einem umlenkbaren Ventilhebel. Das Ventil am freien Ende einer intravaskulären katheterartigen odr schleusenartigen medizinischen Einrichtung zum Zuführen von Arzneimitteln in oder zur steuerbaren Entnahme von Körperflüssigkeiten aus einem humanen oder tierischen Gefäß beispielsweise bei Untersuchungen am Herzen.

In der Medizintechnik ist die Verwendung z.B. von Dreiwegventilen als proximale Endteile von Kathetern oder Schleusen bekannt Wenn an einem der drei Anschlußstutzen eines Dreiwegeventils z.B. eine intravaskuläre Einführungsschleuse in einem Gefäß eines Patienten angeschlossen ist, kann wahlweise in Abhängigkeit von der Stellung des Ventilhebels über den zweiten oder über den dritten Anschlußstutzen je ein Arzneimittel in die Einführungsschleuse zu dem Gefäß abgegeben werden oder es können auch gleichzeitig zwei verschiedene Arzneimittel in die Einführungsschleuse abgegeben werden.

Wenn es sich z.B. um eine Herzkatheteruntersuchung handelt, können gegebenenfalls in unmittelbarer Nähe zusätzlich zu der Einführungsschleuse weitere Katheter und Einführungsschleusen notwendig sein.

Beim Stand der Technik ergibt sich vielfach das Problem, dass in der Ventilabsperrstellung des Dreiwegeventils zur Einführungsschleuse, in der der Ventilhebel üblichwerweise in Richtung der Einführungsschleuse zeigt und in der die Einführungsschleuse aus dem Gefäß zurückgezogen oder ausgewechselt werden kann, der Steuerhebel des Dreiwegeventils sich leicht mit den weiteren im Einsatz befindlichen Kathetern und Einführungsschleusen z.B. bei einer Herzkatheteruntersuchung verhaken oder verklemmen kann, was zu erheblichen Komplikationen führen kann, die z. B. Behandlung am Herzen erheblich gefährden oder behindern kann.

Die DE 10 2007 049 126 A1 zeigt ein Mehrwegeventil mit drei T-förmig zueinander angeordneten Leitungen, welches über ein radial an der mittleren der Leitungen abstehendes Griffelement verstellt werden kann, um ein Fluid entweder über die mittlere Leitung um 90 Grad oder geradlinig über die fluchtenden Leitungen zu leiten. Ist das Ventil so eingestellt, dass das Fluid durch die fluchtenden Leitungen strömen kann, so weist das Griffelement in Richtung der Leitungen und die Unterseite des Griffelements ist nahe an den Leitungen angeordnet, so dass sich eine Schlauchleitung nicht zwischen dem Griffelement und einer der Leitungen verfangen kann.

Die WO 2006/025054 A2 zeigt ein Dreiwege-Ventil mit drei T-förmig zueinander angeordneten Leitungen, welches über einen oben auf dem Ventil angeordneten Ventilhebel verstellt werden kann. Der Ventilhebel kann in vier unterschiedlichen Stellungen angeordnet sein und weist dabei in drei Fällen in Richtung der Leitung, welche versperrt ist.

Die EP 2 168 628 A1 zeigt ein Sicherheitsventil für einen Katheter, welches über einen oben auf dem Ventil angeordneten Ventilhebel verstellt werden kann, wobei der Hebel beidseitig von dem Ventil radial absteht und in Richtung der Leitung weist, wenn das Ventil geöffnet ist. Hinter dem Ventil zwischen Ventil und Operateur ist ein Schild angeordnet.

Aufgabe der Erfindung ist es daher, eine Lösung anzubieten, die ein störungsfreies Zurückziehen oder Wechsel z.B. einer Einführungsschleuse oder eines Katheters in der Absperrstellung zum Dreiwegeventil sicherstellt, in der in gewohnter Weise der Ventilhebel in Richtung der Einführungsschleuse zeigt. Diese Stellung kann der behandelnde Arzt auch bei schwacher Beleuchtung erfühlen, um z.B. sicherzugehen, dass vor einer Zurückziehung oder einem Wechsel der Einführungsschleuse sich das Dreiwegeventil gegenüber der Einführungsschleuse in seiner Abschlußstellung befindet. Beim Verschwenken des Ventilhebels in diese Abschlußstellung kann sich der Ventilhebel leicht mit weiteren Kathetern oder Schleusen, die zusätzlich in unmittelbarer Nähe vorhanden sind, verklemmen oder verhaken, bevor er die volle Abschlußstellung erreicht hat, so dass dann ein Zurückziehen der Einführungsschleuse aus dem Gefäß oder ein Wechsel der Einführungsschleuse aus dem Gefäß oder ein Wechsel der Einführungsschleuse erschwert oder blockiert ist.

Die Aufgabe wird erfindungsgemäß mit den Merkmalen des Anspruchs 1 gelöst. Vorteilhafte Ausführungen des im Anspruch 1 angegebenen Erfindungsgegenstandes sind den Unteransprüchen und der nachfolgenden Beschreibung zu entnehmen.

Die Erfindung wird anhand der nachstehenden Zeichnungen beschrieben und erläutert. Hierzu zeigt:
- Fig. 1a: ein konventionelles Dreiwegeventil z.B. in Verbindung mit einer Einführungsschleuse in der Draufsicht;
- Fig. 1b: das konventionelle Dreiwegeventil nach Fig. 1a in der Seitenansicht;
- Fig. 2: ein Dreiwegeventil gemäß der Erfindung in einer ersten Ausführung in der Seitenansicht;
- Fig. 3: ein Dreiwegeventil gemäß der Erfindung in einer zweiten Ausführungsform in der Seitenansicht;
- Fig. 4: ein Dreiwegeventil gemäß der Erfindung in einer dritten Ausführungsform in der Seitenansicht;
- Fig. 5: ein Dreiwegeventil gemäß der Erfindung in einer vierten Ausführungsform in der Seitenansicht und
- Fig. 6: ein Dreiwegeventil gemäß der Erfindung in einer fünften Ausführungsform in der Ansicht von vorne auf den Anschlußstutzen des Ventils zum Anschluß an die nicht gezeigte Einführungsschleuse.

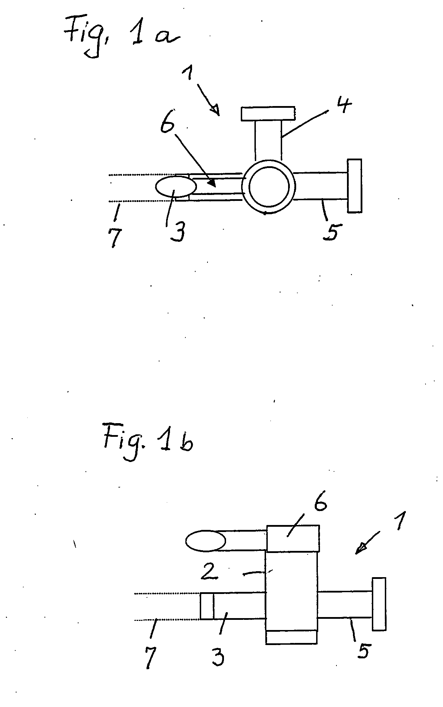

In Fig. 1a und 1b ist ein herkömmliches Dreiwegeventil in der Draufsicht (Fig. 1a und in der Seitenansicht (Fig. 1b) mit 1 bezeichnet. Das zylindrische Ventilgehäuse ist mit 2 bezeichnet, von dem drei radiale Anschlußstutzen 3, 4 und 5 ausgehen, die jeweils einen Winkel von 90° zueinander aufweisen. In dem zylindrischen Ventilgehäuse 2 ist ein nicht näher dargestellter bekannter zylindrischer Ventilkörper mit einem T-förmigen Steuerkanal drehbar gelagert, wobei der Ventilkörper das axial obere Ende des Ventilgehäuses 2 etwas überragt, an dem ein radial sich nach außen erstreckender Ventilhebel 6 in vorgegebener Länge zum Verdrehen des Ventilkörpers drehfest angeschlossen ist.

In Fig. 1a weist der verschwenkbare Ventilhebel 6 radial in Richtung der hier beispielsweise verwendeten nicht weiter dargestellten Einführungsschleusen, die mit Ventilanschlußende 7 an den einen verlängerten Anschlußstutzen 3 des Dreiwegeventils 1 fest angeschlossen ist.

An die beiden anderen Anschlußstutzen 4 und 5 ist jeweils ein nicht dargestellter Zuführungsleiter für Arzneimittel lösbar angeschlossen.

Durch Drehung des Ventilhebels 6 mit dem Uhrzeigersinn aus seiner in Fig. 1a und 1b gezeigten Schließstellung gegenüber der Einführungsschleuse 7 um 90° in Richtung des Anschlußstutzens 4 wird dieser Anschlußstutzen abgesperrt und zugleich der Anschlußstutzen 5 über den offenen Anschlußstutzen 3 an die Einführungsschleuse 7 angeschlossen.

Durch eine weitere Drehung des Ventilhebels 6 im Uhrzeigersinn um 90° weist der Ventilhebel 6 in Richtung des Anschlußstutzens 5, in der dieser abgesperrt ist und dafür der Anschlußstutzen 4 an den offenen Anschlußstutzen 3 zur Einführungsschleuse 7 angeschlossen ist.

Durch ein Weiterdrehen des Ventilhebels 6 um 90° in eine Richtung entgegengesetzt zum Anschlußstutzen 4 sind beide Anschußstutzen 4 und 5 an den offenen Anschlußstutzen 3 angeschlossen.

Durch die Wahl der Stellung des Ventilhebels 6 wird entweder. Arzneimittel über den Anschlußstutzen 4 und/oder 5 in die Einführungsschleuse 7 abgegeben.

Wenn der Ventilhebel 6 in die Richtung eines der Anschlußstutzen 3, 4 oder 5 weist, dann zeigt das -wie gesagt - dem behandelnden Arzt die Abschlußstellung (Schließstellung) des betreffenden Anschlußstutzen 3 bzw. 4 bzw. 5 an.

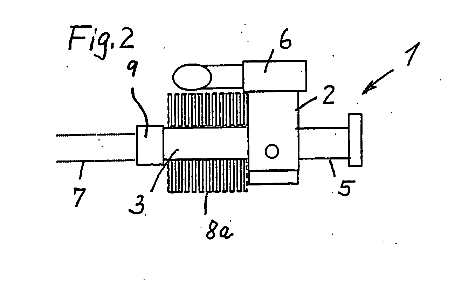

In den Figuren 2 bis 6, in denen ohne jede Beschränkung 5 Ausführungsbeispiele für das Dreiwegeventil nach der Erfindung gezeigt sind, sind entsprechende Ventilteile mit den gleichen Bezugszeichen bezeichnet, wie sie in den Fig 1 a und 1b verwendet sind.

Die Vorrichtungen an dem erfindungsgemäßen Dreiwegeventil zum störungsfreien Zurückziehen der Einführungsschleuse 7 aus dem behandelten Gefäß oder zum Wechsel der Einführungsschleuse 7 sind jeweils mit dem Bezugszeichen 8 als Grundzeichen bezeichnet, das durch Indices a, b, c, d und e unterschieden ist.

In Fig. 2 besteht die Sperrvorrichtung aus einem an den Durchmesser des verlängerten Anschlußsstutzens 3 angepassten Ringkörper 8a, der über den Anschlußstutzen 3 des Dreiwegeventils 1 bis zum Anschlag an das Ventilgehäuse 2 geschoben ist. Der Ringkörper 8a weist einen radialen Durchmesser und eine solche axiale Breite auf, dass der freie Spalt zwischen der Unterseite des Ventilhebels 6 und der Oberseite des Anschlußstutzens 3 des Dreiwegeventils 1 weitgehend abgeschlossen ist, wobei die freie Verschwenkbarkeit des Ventilhebels nicht behindert ist.

Die ringförmige Sperrvorrichtung 8a kann an dem verlängerten Anschlußstutzen 3 fest angeschlossen sein. Sie kann aber auch auswechselbar auf den Anschlußstutzen 3 aufgeschoben sein.
Gegen ein ungewolltes Abziehen der Sperreinrichtung 8a von dem Anschlußstutzen 3 ist am vorderen Ende des Anschlußstutzens eine Sicherungsmuffe 9 vorgesehen, die gegebenenfalls auch entfallen kann.

Die Sperrvorrichtung 8a kann aus beliebigem, die hier notwendige Sperrfunktion erfüllenden Material, insbesondere aus Kunststoff, bestehen, der sich in vergleichbaren medizinischen Geräten bewährt hat.

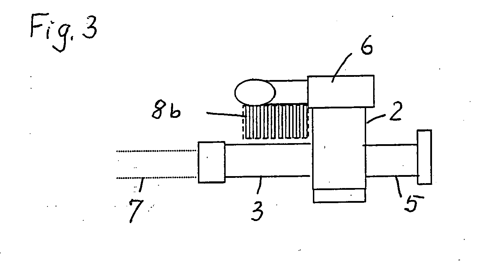

In Fig. 3 besteht die Sperrvorrichtung 8b aus einem streifenförmigen Material, das hier z.B. an der Unterseite des Ventilhebels 6 fest angeordnet ist und in der Schließstellung den Ventilhebel 6 zum Abschluß des Anschlußstutzens 3 den freien Spalt zwischen der Unterseite des Ventilhebels 6 bis dicht an die Oberseite des Anschlußstutzens 3 ausfüllt, wobei die freie Verschwenkbarkeit des Ventilhebels nicht eingeschränkt wird. Entsprechend könnte die Sperrvorrichtung auch an der Oberseite des Anschlußstutzens 3 angeordnet sein.

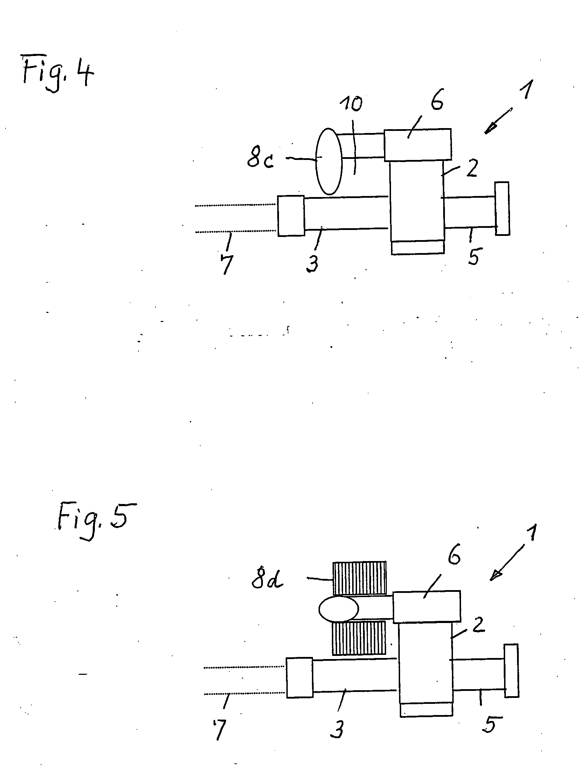

In Fig. 4 ist die Sperrvorrichtung 8c aus einem schildartigen Teil ausgebildet, der am stirnseitigen Ende des Ventilhebels 6 fest oder lösbar angeordnet ist und dabei das obere Ende des Ventilhebels nur geringfügig überragt aber sich mit seinem unteren Ende im rechten Winkel zum Ventilhebel 6 senkrecht nach unten bis nahe an die Außenfläche des Anschlußstutzens 3 erstreckt. Hierdurch wird der freie Spalt 10 zwischen der Unterseite des Ventilhebels 6 und der Oberseite des Anschlußstutzens 3 von außen sicher abgeschlossen, um ein Verhaken oder Verklemmen mit dem Ventilhebel 6 mit zusätzlichen Schleusen oder Kathetern sicher zu vermeiden.

Fig. 5 zeigt eine weitere Variante der Sperrvorrichtung 8d, die vergleichbar mit der Lösung in Fig. als Ringkörper ausgebildet ist, der mit seiner zentralen Öffnung über einen radialen Abschnitt des Ventilhebels 6 geschoben ist und hier fest oder abziehbar auf dem Ventilhebel 6 angeordnet ist. Der Ringkörper 8d weist hier einen kleinen Abstand von dem Ventilgehäuse 2 auf, um eine unbehinderte Schwenkbewegung des Ventilhebels 6 aufrecht zu erhalten.

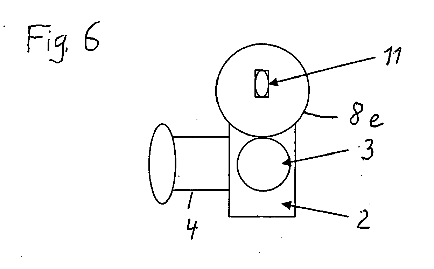

Die Sperrvorrichtung nach Fig. 6, besteht aus einem ringförmigen Scheibenkörper 8e, der eine zentrale Öffnung 11 zum Aufstecken des scheibenartigen Sperrkörpers 8e auf das vordere Ende des Ventilhebels 6 aufweist, wodurch vergleichbar mit der Lösung in Fig. 4 der freie Spalt 10 zwischen der Unterseite des Ventilhebels 6 und der Oberseite des Anschlußstutzens 3 in einer Weise dicht absperrt ist, dass ein Verhaken oder Verklemmen mit zusätzlichen Schleusen und/oder Kathetern sicher vermieden ist.

Auch hier kann der ringförmige Scheibenkörper 8e mit dem äußeren Ende des Ventilhebels fest verbunden sein oder auch lösbar am vorderen Ende des Ventilhebels 6 aufgesteckt sein.

Das schildartige Teil 8c in Fig. 4 und der ringförmige Scheibenkörper 8e in Fig. 6 jeweils am vorderen Ende des Ventilhebels 6 können mit diesem auch einstückig sein.

Die vorstehenden Varianten für die Sperrvorrichtungen 8 sind entweder am verlangerten Anschlußstutzen 3 für die Einführungsschleuse 7 oder am Ventilhebel 6 fest oder lösbar angeordnet. Der hier betreffende Fachmann erhält durch diese Ausführungen der Erfindung ohne weiteres Anregungen zu weiteren Varianten im Rahmen des vorliegenden Erfindungsgedankes zur störungsfreien Handhabung beispielsweise eine Einführungsschleuse, die mit einem Dreiwegventil fest verbunden ist.

Mit den erfindungsgemäßen Sperrvorrichtungen ist jeweils sicher gestellt, dass bei einem Zurückziehen z.B. einer Einführungsschleuse aus einem Gefäß oder bei einem Wechsel der Einführungsschleuse der Ventilhebel 6 des Dreiwegeventils 1 mit weiteren Kathetern oder Einführungsschleusen nicht verklemmt oder verhakt ist, so dass das Zurückziehen oder der Wechsel der Einführungsschleuse erschwert oder blockiert ist und erst nach einer Zeitverzögerung ermöglicht ist, nachdem die Verklemmung oder Verhakung mit den weiteren Schleusen und Kathetern aufgelöst ist.

## Patentansprüche

1. Dreiwegeventil (1) mit drei Anschlußstutzen (3, 4 und 5) und einem Ventilhebel (6), wobei einer der Anschlußstutzen (3) verlängert ist und über eine Einführungsschleuse (7) und an ein Gefäß eines Patienten oder zur Entnahme von Körperflüssigkeit oder zur Zuführung von Arzneimitteln lösbar dicht anschliessbar ausgebildet ist,
**dadurch gekennzeichnet,**
**dass** zur Erhöhung der Behandlungssicherheit mit dem Dreiwegeventil am Patienten der freie Zutritt in einen offenen Spalt (10), der zwischen der Oberseite des verlängerten Abschlußstutzens (3) und dem vorderen Ende an der Unterseite des Ventilhebels (6) mit der hinteren Spaltbegrenzung durch den Ventilkörper (2) gebildet ist, durch
eine Sperrvorrichtung (8a) am verlängerten Abschlußstutzen (3) oder
eine Sperrvorrichtung (8b, 8d) am Ventilhebel (6) absperrbar ausgebildet ist oder der freie Zutritt in den offenen Spalt (10) mittels eines ringförmigen Körpers (8e) am vorderen Ende des Ventilhebels (6) versperrt ist.

2. Ventil (1) nach Anspruch 1, **dadurch gekennzeichnet, dass** die Sperrvorrichtung als Ringkörper ausgebildet ist.

3. Ventil nach Anspruch 2, **dadurch gekennzeichnet, dass** der Ringkörper fest oder abziehbar auf dem Ventilhebel (6) angeordnet ist.

4. Ventil nach Anspruch 1, **dadurch gekennzeichnet, dass** die Sperrvorrichtung (8) aus einem streifenförmigen Material oder als schildartiger Teil oder als ringförmiger Scheibenkörper ausgebildet ist.

5. Ventil nach einem der vorhergehenden Ansprüche, **dadurch gekennzeichnet, dass** die Sperrvorrichtung (8) aus Kunststoff ausgeführt ist.

## Claims

1. A three-way valve (1) with three connection fittings (3, 4, and 5) and a valve lever (6), in which one of the connection fittings (3) is elongated and is embodied so that it can be detachably connected in a sealed fashion via a catheter (7) to a vessel of a patient either for withdrawing bodily fluid or administering drugs,
**characterized in that**
in order to increase the treatment safety with the three-way valve in the patient, the free entry into an open gap (10), which is formed between the top of the elongated connection fitting (3) and the front end on the bottom of the valve lever (6) with the rear boundary of the gap being formed by the valve body (2),
is embodied so that it can be blocked by a blocking device (8a) on the elongated connection fitting (3) or by a blocking device (8b, 8d) on the valve lever (6) or else the free entry into the open gap (10) is blocked by means of an annular body (8e) at the front end of the valve lever (6).

2. The valve (1) according to claim 1, **characterized in that** the blocking device is embodied in the form of an annular body.

3. The valve according to claim 2, **characterized in that** the annular body is permanently or detachably mounted to the valve lever (6).

4. The valve according to claim 1, **characterized in that** the blocking device (8) is composed of a strip-shaped material or is embodied in the form of a shield-like part or an annular disc body.

5. The valve according to one of the preceding claims, **characterized in that** the blocking device (8) is made of plastic.

## Revendications

1. Vanne à trois voies (1) comportant trois tubulures de raccordement (3, 4 et 5) et un levier de vanne (6), l'une des tubulures de raccordement (3) étant prolongée et réalisée de manière à pouvoir être raccordée de façon étanche et détachable via une gaine d'introduction (7) et sur un vaisseau d'un patient ou pour prélever un liquide corporel ou pour administrer des médicaments,
**caractérisée en ce que**
en vue d'augmenter la sécurité de traitement avec la vanne à trois voies pour le patient, l'accès libre à une fente ouverte (10) formée entre le côté supérieur de la tubulure de raccordement prolongée (3) et l'extrémité avant sur le côté inférieur du levier de vanne (6) avec la délimitation de fente arrière par le corps de vanne (2), est réalisé de manière a pouvoir être obturé par un dispositif obturateur (8a) sur la tubulure de raccordement prolongée (3) ou par un dispositif obturateur (8b, 8d) sur le levier de vanne (6), ou bien l'accès libre dans la fente ouverte (10) est obturé au moyen d'un corps annulaire (8e) à l'extrémité avant du levier de vanne (6).

2. Vanne (1) selon la revendication 1, **caractérisée en ce que** le dispositif obturateur est réalisé sous forme de corps annulaire.

3. Vanne selon la revendication 2, **caractérisée en ce que** le corps annulaire est agencé de façon fixe ou retirable sur le levier de vanne (6).

4. Vanne selon la revendication 1, **caractérisée en ce que** le dispositif obturateur (8) est réalisé à partir d'un matériau en forme de ruban ou sous la forme d'un élément formant bouclier ou sous la forme d'un corps de rondelle annulaire.

5. Vanne selon l'une des revendications précédentes, **caractérisée en ce que** le dispositif obturateur (8) est réalisé en matière plastique.
